# EUROPEAN PATENT SPECIFICATION

(11) **EP 3 592 815 B1**
(45) Date of publication and mention of the grant of the patent: **25.10.2023**
(21) Application number: 18709014.7
(22) Date of filing: 08.03.2018
(51) Int. Cl.: C09D 5/18, C08G 77/04

(54) **THERMAL INSULATION MATERIALS**
WÄRMEISOLATIONSMATERIAL
MATERIAUX D'ISOLATION THERMIQUE

(30) Priority: 08.03.2017 EP 17159963
(43) Date of publication of application: 15.01.2020
(73) Proprietor: Silana GmbH, 8702 Zollikon (CH)
(72) Inventor: SEEGER, Stefan, 8702 Zollikon (CH)
(74) Representative: Schmitz, Joseph
(86) International application number: PCT/EP2018/055781
(87) International publication number: WO 2018/162648

(56) References cited:
- WO-A1-2016/001377
- WO-A2-2004/113456
- PRZYBYLAK MARCIN ET AL: "Multifunctional, strongly hydrophobic and flame-retarded cotton fabrics modified with flame retardant agents and silicon compounds", POLYMER DEGRADATION AND STABILITY, BARKING, GB, vol. 128, 4 March 2016 (2016-03-04), pages 55-64, XP029528393, ISSN: 0141-3910, DOI: 10.1016/J.POLYMDEGRADSTAB.2016.03.003

## Description

### TECHNICAL FIELD

The present invention relates to thermal insulation materials, and in particular thermal insulation materials that can be renewably sourced from natural materials, comprising a flame retardant coating as well as to a method of manufacturing such thermal insulation materials.

### PRIOR ART

Thermal insulation is the reduction of heat transfer (the transfer of thermal energy between objects of differing temperature) between objects in thermal contact and having different temperatures. Thermal insulation materials provide a region of insulation in which thermal conduction is reduced rather than unhindered heat transfer towards the lower-temperature body.

Thermal insulation materials can be used in multiple applications ranging from inner insulation layers in garments that reduce the loss of heat from the wearer towards the environment to forming the thermal envelope of a building in order to either prevent the loss of heat from the inside of the building in cold climates or the loss of cold from the inside of the building in warm climates.

Thermal insulation materials come in a multitude of forms but in general are materials having a density which is inferior to 500 kg/m³, since the insulating effect derives from the fact that the air, which is in itself a good thermal insulator, is immobilized in the interstices or cavities of the thermal insulation material such as to prevent heat transfer through convection. Thus, there is a tendency to minimize the thermal insulation material while maximizing the air enclosed in it. This, however, creates new collateral problems such a flammability of the thermal insulation material, since the lower density thermal insulation materials offer a high surface to weight ratio, thereby exposing a large surface of the thermal insulation material to ignition and on the other hand enclosing large amounts of air capable of, once ignited, fueling the combustion of the thermal insulation material.

The above problem is further exacerbated by the recent trend of replacing inorganic insulation materials that have been deemed to be hazardous to human health with renewably sourced materials in the manufacture of thermal insulation materials, such as wood wool or cellulose fiber, since these materials are more flammable when compared to inorganic insulation materials. In addition, such renewably sourced materials are subject to decomposition by fungi or microorganisms whose growth is promoted by moisture which may condense on the surface of the thermal insulation materials and then wick into them. This is of particular nuisance since once started, the decomposition can progress unnoticed as in most cases, the thermal insulation material is hidden from sight when installed.

"Multifunctional, strongly hydrophobic and flame-retarded cotton fabrics modified with flame retardant agents and silicon compounds" in Polymer Degradation and Stability 128 (2016) 55-64 discloses a sol-gel process for flame retardant modification of cotton fibers in which cotton fibers are exposed to 3-aminopropyltriethoxysilane (APS) and a flame retardant agent and the cotton fibers are thereby coated with a multidimensional polysiloxane network that incorporates the flame retardant agent, thus conferring flame retardant properties to the cotton fiber. However, the thus obtained flame retardant modification is not water-repellent and can therefore absorb moisture which in turn might lead to leaching out of the flame retardant and to decomposition of the cotton. This is remediated by applying a second, separate modification of fluorofunctional silanes or fluorofunctional siloxanes to the already flame retardant cotton fiber. In sum, such a process requires two interdependent discrete steps to arrive at a thermal insulation material that incudes still includes potentially problematic flame retardant agents and does not yield a coating having a nanofibrillar surface structure.

### SUMMARY OF THE INVENTION

It is a first object of the present invention to provide a use of a coating comprising nano-filaments obtained by a polymerization reaction of one or more silane compounds in the presence of water as a flame retardant coating applied on a surface of a material such as a thermal insulation material or medical textiles. The thermal insulation material that has a coating applied thereof and which coating confers improved flame resistance to said thermal insulation material when compared to the uncoated material. Furthermore, the thermal insulation material according to the present invention has good decomposition resistance due to fungal and microbial colonization. The thermal insulation material according to the present invention comprises a flame retardant coating applied on a surface thereof, characterized in that the flame retardant coating comprises nano-filaments obtained by a polymerisation reaction of one or more silane compounds in the presence of water. Without wishing to be bound to a certain theory, it is believed that in addition to offering protection against flames because of the coating's chemical composition, the nanofilament surface morphology provides additional flame resistance because the thermal insulation material cannot be easily reached by the flame and consequently, the ignition of the thermal insulation material is delayed.

In a preferred embodiment of the thermal insulation material according to present invention, the one or more silane compounds is chosen from alkylsilanes, alkenylsilanes, arylsilanes or derivatives thereof, and in particular from silane compounds of the formula I

RₐSi(R¹)ₙ(X¹)₃₋ₙ I

wherein
Rₐ is a straight-chain or branched C₁₋₂₄ alkyl group or an aromatic group which is linked by a single covalent bond or a spacer unit to the Si- atom,
R¹ is a lower alkyl group,
X¹ is a hydrolysable group, and
n is 0 or 1, with the proviso that X¹ may represent the same or different groups.

In another preferred embodiment of the thermal insulation material according to present invention, the polymerisation reaction of one or more silane compounds in the presence of water is carried out in the gas phase under conditions such that the relative humidity is in the range of 20 % to 80%, preferably in the range of 30% to 60% and most preferably in the range of 30% to 50%.

In another preferred embodiment of the thermal insulation material according to present invention, the thermal insulation material according is a fibrous material preferably in the form of a non-woven fibre batt or a non-woven fabric such as spunbond or flashspun non-woven fabric.

In another preferred embodiment of the thermal insulation material according to present invention, the thermal insulation material has a density of from 10 to 350 kg/m³, preferably 25 to 250 kg/m³.

In another preferred embodiment of the thermal insulation material according to present invention, the thermal insulation material is sourced from a renewable raw material such as wood wool, recycled wood fiber boards, straw, hemp, reed, grass, flax, or animal wool, or feathers.

In another preferred embodiment of the thermal insulation material according to present invention, is sourced from inorganic raw materials such as glass and stone.

In another preferred embodiment of the thermal insulation material according to present invention, is sourced from synthetic polymer raw materials such as polyester, polyamide, polyethylene or polypropylene.

In a preferred embodiment of the use of a coating comprising nano-filaments as a flame retardant coating according to present invention, the one or more silane compound is chosen from alkylsilanes, alkenylsilanes, arylsilanes or derivatives thereof, in particular from silane compounds of the formula I:

RₐSi(R¹)ₙ(X¹)₃₋ₙ I

wherein
Rₐ is a straight-chain or branched C₁₋₂₄ alkyl group or an aromatic group which is linked by a single covalent bond or a spacer unit to the Si- atom,
R¹ is a lower alkyl group,
X¹ is a hydrolysable group, and
n is 0 or 1, with the proviso that X¹ may represent the same or different groups.

In another preferred embodiment of the use of a coating comprising nano-filaments as a flame retardant coating according to present invention, the material is a thermal insulation material.

In another preferred embodiment of the use of a coating comprising nano-filaments as a flame retardant coating according to present invention, the material is a textile.

In another preferred embodiment of the use of a coating comprising nano-filaments as a flame retardant coating according to present invention, the material is a medical dressing or medical bandage.

It is a further object to provide a thermal insulation panel incorporating thermal insulation material such as for example wood wool according to the first object of the present invention comprising a flame retardant coating applied on a surface thereof, characterized in that the flame retardant coating comprises nano-filaments obtained by a polymerisation reaction of one or more silane compounds in the presence of water.

Further embodiments of the invention are laid down in the dependent claims.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: shows on the left, a sequence of photographs of a virgin wood wool sample after being exposed to a source of ignition, from top to bottom, as well as a sequence of photographs of a wood wool sample having a flame retardant coating according to the present invention, on the right, after being exposed to a source of ignition, from top to bottom.

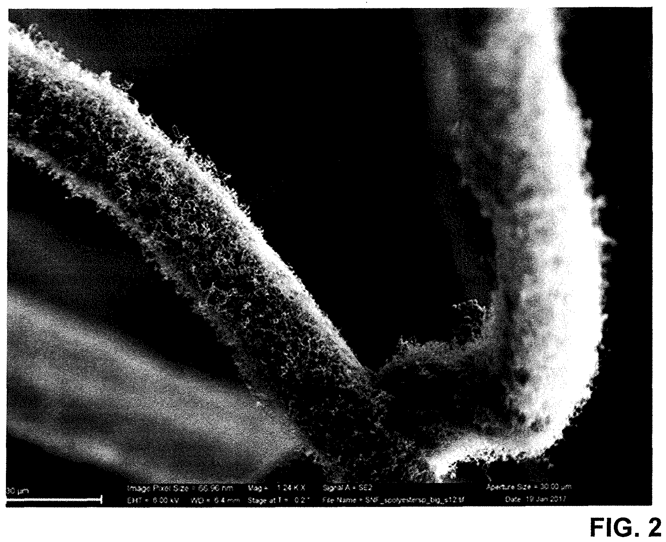

- Fig. 2: shows a scanning electron microscope 1.24 K magnification of a polyester filament having a flame retardant coating of nano-filaments obtained by gas phase polymerisation attached to its surface.

### DESCRIPTION OF PREFERRED EMBODIMENTS

The thermal insulation material according to the present invention comprises a flame retardant coating applied on a surface thereof, characterized in that the flame retardant coating comprises nano-filaments obtained by a polymerisation reaction of one or more silane compounds in the presence of water.

The flame retardant coatings resulting from the polymerisation reaction of one or more silane compounds in the presence of water exhibit a special nanofilament morphology, which the inventors believe to be at the root of the conferred flame resistance. The nanofilaments formed have a diameter of about 10 to 160 nm and a length of about 2, 3 or more micrometres. While the morphology is in general that of nanofilaments, it has also been observed that they can have a beads-on-a-string type morphology, depending on the type of silane and water concentration used.

The one or more silane compounds suitable in the production of the coatings can be any type of silane, provided the silane includes at least one hydrolysable group and preferably at least one hydrolysable group and at least two non-hydrolysable groups such as alkyl, alkylene, alkylaryl and aryl groups. The hydrolysable group can preferably be a halide such as chlorine or bromine, or an alkoxy group such as for example methoxy or ethoxy groups.

The coatings may be exclusively obtained by polymerisation reaction of one or more silane compounds in the presence of water without the addition of further flame retardants and may further be free from phosphorus- and/or nitrogen-containing compounds.

In general, the thermal insulation material is in a fibrous form such as filaments, fibres or shavings and is then further processed into all sorts of webs such as slivers, batts, blankets, loose-fill fibre, felts, spun-bond or flash spun non-wovens and fibre panels. The flame retardant coating can be applied either to the unprocessed or to the processed thermal insulation material such as for example fibre batts.

Spun-laid, also called spun-bond, nonwovens are made in one continuous process. Fibers are spun and then directly dispersed into a web by deflectors or can be directed with air streams. The can generally be made from polyolefins such as PP or polycondensates such as polyester or polyamide.

In a preferred embodiment the thermal insulation material is a spun-bond non-woven that has been combined with melt-blown non-woven, conforming them into a layered product called SMS (spun-melt-spun). Melt-blown nonwovens have extremely fine fiber diameters but are not strong fabrics which are then bonded to spun-bonded non-wovens by either resin or thermally.

In general, the thermal insulation material can be sourced from a renewable material such as plant material or animal material. Suitable plant material can be softwood or hardwood, grass, straw, cotton whereas suitable animal material can be wool such as sheep wool. While in some cases, the source is already in fibrous form, such as with wool, in other cases the source must be brought into fibrous form to provide the adequate low density for use as a thermal insulation material. For instance, in the case of wood, the wood can be cut into wood wool or the wood may be chemically transformed into a cellulosic or lingo-cellulosic fibre such as viscose.

In general, the thermal insulation material can also be sourced from materials which are usually used in the manufacture of thermal insulation material such as inorganic materials. Such inorganic materials can be chosen from glass, silicate, rock and other minerals.

The one or more silane compounds useful for obtaining the flame retardant coating can in general be chosen from compounds of formula I when using one silane

RₐSi(R¹)ₙ(X¹)₃₋ₙ I

wherein
- Rₐ: is a straight-chain or branched C₁₋₂₄ alkyl group or an aromatic group which is linked by a single covalent bond or a spacer unit to the Si- atom,
- R¹: is a lower alkyl group,
- X¹: is a hydrolysable group, and
- n: is 0 or 1, with the proviso that X¹ may represent the same or different groups.

Alternatively, when using two or more silanes, the compounds useful for obtaining the flame retardant coating can in general be chosen from compounds of formula I and at least one compound of formula II

R^{a}Si(R¹)ₙ(X¹)₃₋ₙ I

R^{b}Si(R²)ₘ(X²)₃₋ₘ II

wherein
- Rₐ: is a straight-chain or branched C₍₁₋₂₄₎ alkyl group,
- R^{b}: is an aromatic group which is linked by a single covalent bond or a spacer unit to the Si- atom,
- R¹ and R²: are independently of each other a lower alkyl group,
- X¹ and X²: are independently of each other a hydrolysable group, and
- n, m: are independently of each other 0 or 1,
with the proviso that if n and m are independently of each other 0 or 1, X may represent the same or different groups.

It is understood that the term "straight-chain or branched C₍₁₋₂₄₎ alkyl group" includes preferably straight chain and branched hydrocarbon radicals having 1 to 16, more preferably 1 to 12, more preferably 1 to 8 carbon atoms and most preferred 1 to 4 carbon atoms, such as methyl, ethyl, n-propyl, isopropyl, n-butyl and isobutyl groups.

It is understood that the term "aromatic" includes optionally substituted carbocyclic and heterocyclic groups comprising five-, six-or ten-membered ring systems, such as furane, phenyl, pyridine, pyrimidine, or naphthalene, preferably phenyl, which are unsubstituted or substituted by an optionally substituted lower alkyl group, such as methyl, ethyl or trifluoromethyl, a halogen, such as fluoro, chloro, bromo, preferably chloro, a cyano or nitro group.

It is understood that the term "spacer unit" includes a straight-chain or branched alkyl residue, having 1 to 8 carbon atoms, preferably 1 to 6, more preferably 1, 2 or 3 carbon atoms.

It is understood that the term "lower alkyl" includes straight chain and branched hydrocarbon radicals having 1 to 6 carbon atoms, preferably 1 to 3 carbon atoms. Methyl, ethyl, propyl and isopropyl groups are especially preferred.

It is understood that the term "hydrolysable group" includes a halogen, such as fluoro or chloro, preferably chloro, or an alkoxy group, such as a straight chain and branched hydrocarbonoxy radical having 1 to 6 carbon atoms, preferably 1 to 3 carbon atoms, wherein methoxy, ethoxy, propoxy and isopropoxy groups are especially preferred.

In both cases, particularly preferred examples of compounds of formula I include trichloromethylsilane (TCMS), trichloroethylsilane, trichloro(n-propyl)silane, trimethoxymethylsilane and triethoxymethylsilane and when using two or more silanes, particularly preferred examples of compounds of formula II include (3-phenylpropyl)-methyldichlorosilane (PMDS), benzyltrichlorosilane, methylbenzyltrichlorosilane and trifluoromethylbenzyltrichlorosilane.

In case of acid-sensitive substrates it is preferred to use alkoxysilanes, such as methyltriethoxysilane, (3-phenylpropyl)-methyldimethoxysilane or (3-phenylpropyl)-methyldiethoxysilane, to avoid the formation of hydrochloric acid during hydrolysis of the silanes with the water molecules in the reaction volume or at the substrate surface.

If the flame retardant coating comprises a compound of formula II, the volume ratio of compound of formula I to compound of formula II ranges from 1:100 to 100:1, preferably from 1:50 to 50:1, more preferably from 1:10 to 10:1, most preferably from 1:1 to 5:1 depending on the nature of the compounds and the nature of the substrate. For example, on inorganic thermal insulation materials such as glass wool, a composition comprising TCMS and PMDS in a volume ratio of 3:1 is preferred.

The flame retardant coating is preferably applied in the gas phase, since in the gas phase the silanization mixture of one or more silanes and water can penetrate into the thermal insulation material easily and more in-depth silanization can be achieved. On a smaller scale, a simple desiccator may be used as reaction vessel for the silanization. The one or more silane is placed in a closed Eppendorf tube, which is fixed in a special holder. The holder comprises a mechanism for opening the Eppendorf tube which can be triggered from outside by a magnet. The desiccator holding the Eppendorf tube and the uncoated thermal insulation material is closed and flushed by a suitable carrier gas, e.g. a nitrogen/water gas mixture. The relative humidity of the gas mixture needed in the desiccator can be set by independently adjusting the flow rates of dry and wet gas stream by two valves combined with rotameters. The gas streams are mixed in a mixing chamber where the relative humidity is controlled by a hygrometer, and may for example be set in general to about 30 to 60% to form filaments. The desiccator is then flushed until the relative humidity measured by a second hygrometer at the outlet of the desiccator remains constant and corresponds to the set value. The inlet and outlet cocks at the desiccator are then closed and the coating reaction is started by opening the Eppendorf tube. Depending on the volatility of the silanes, the reaction may be run at atmospheric pressure or lower pressures if necessary. The reaction is completed within 0 to 24 hours and typically after twelve hours. After rinsing with an aqueous solvent, such as water, the coated insulation material is ready for use.

As a final step the coated material may optionally be submitted to a curing step to complete the condensation reaction of remaining free hydroxyl groups at the surface of the material and the coating, thereby further increasing the mechanical stability of the flame retardant coating by forming additional cross-linking Si-O-Si bonds within the coating or from the material to the coating.

Alternatively, the silanization may be achieved in solution, either by direct contact with the material in solution or by first polymerizing the one or more silane in solution in the absence of the material and applying the resulting dispersion of nanofilaments onto the material. In the former case, the material is placed at room temperature under stirring in a previously prepared solution comprising the one or more silanes dissolved or suspended in an aprotic solvent, such as toluene in the presence of 5 to 500 ppm, preferably 60 to 250 ppm, more preferably 75 to 150 ppm and most preferably 130 to 150 ppm, of water. After 3 to 4 hours the material is removed, rinsed with for example ethanol and subsequently water and finally dried. In the case of first polymerizing the one or more silane in solution in the absence of the material, a liquid coating composition comprising a solvent and dispersed silicone nanofilaments, preferably in an amount of from 0.01 % to 40% by weight based on the total weight of the liquid coating composition, is formed and then applied as a layer of the liquid coating composition on the surface of the material on which the flame retardant coating is to be formed, and the solvent from the liquid coating composition is evaporated to form the flame retardant coating and impart said property on said surface of the material. The dispersed silicone nanofilaments are formed by introducing total one or more silanes in an aprotic solvent such as toluene comprising 5 to 500 ppm, preferably 60 to 250 ppm, more preferably 75 to 150 ppm and most preferably 130 to 150 ppm, of water.

Characterization of the surface coatings of the invention by scanning electron microscopy, transmission electron microscopy and scanning force microscopy demonstrated the formation of distinct geometrical forms, such as nanofilaments giving rise to the required surface roughness. The fibres are solid and ranged from very short, nearly spherical bases of at least 200 nm in length up to several, i.e 2, 3 or more µm in length with diameters ranging from approximately 10 nm to 160 nm and up to 200 nm.

Such unexpected formation of the surface roughness during condensation reaction as a consequence of self-organisation, i.e. self-arrangement, or self-assembly of the silanes of the present invention is a great advantage over many other coating methods, which do not yield the nano-filamentous morphology as in the present invention.

### EXAMPLES

Fibres were positioned on the surface of the glass slides and anchored with glue, in order to obtain a fibre layer as homogeneous as much as possible. The glass slides, on which are positioned the three materials on three circular tapes (12mmØ, 113mm²) are placed into the desiccator and exposed to gaseous phase silanization to form silicone nanofilaments on the surface thereof. The same procedure was used on fibre wads.

The gaseous phase silanization is realized under a controlled atmosphere with the relative humidity set to 36±0.5%, at room temperature and pressure and left to proceed overnight. For glass fibre based materials, the reaction carried out using 300µl TCMS (trichloromethylsilane)/339 mm² for glass fibres and for wood fibre based materials, the reaction was carried out using 500µl TCMS/339 mm². When silanizing the fibre wads, the amount of silane used was increased 600µl and 1ml, respectively, because the surface area was approximately double that of the glued fibre.

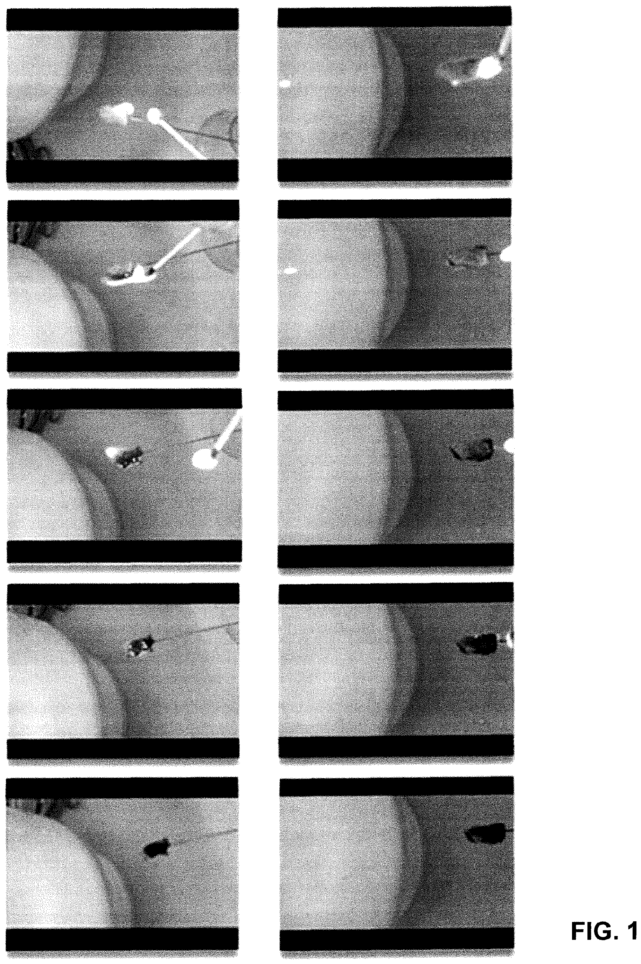

As can be seen from the photograph sequence in the Figure 1, the virgin wood wool wad continues burning after being ignited until essentially all of the wood wool was combusted. On the other hand, the wood wool wad that had been treated with TCMS at relative humidity set to 36±0.5% did not ignite even after prolonged exposure to the flame and did therefore not sustain combustion of the wood wool.

## Claims

1. Use of a coating comprising nano-filaments obtained by a polymerisation reaction of one or more silane compounds in the presence of water as a flame retardant coating applied on a surface of a material.

2. The use according to claim 1, wherein the one or more silane compound is chosen from alkylsilanes, alkenylsilanes, arylsilanes or derivatives thereof, in particular from silane compounds of the formula I:
RₐSi(R¹)ₙ(X¹)₃₋ₙ I
wherein
Rₐ is a straight-chain or branched C₁₋₂₄ alkyl group or an aromatic group which is linked by a single covalent bond or a spacer unit to the Si- atom,
R¹ is a straight chain or branched hydrocarbon radical having 1 to 6 carbon atoms,.
X¹ is a hydrolysable group, and
n is 0 or 1, with the proviso that X¹ may represent the same or different groups.

3. The use according to claim 1 or 2, wherein the polymerisation reaction of one or more silane compounds in the presence of water is carried out in the gas phase and the relative humidity is in the range of 20 % to 80 %, preferably in the range of 30 % to 60 % and most preferably in the range of 30 % to 50 % or is carried out in the liquid phase in an aprotic solvent, such as toluene, in the presence of 5 to 500 ppm, preferably 60 to 250 ppm, more preferably 75 to 150 ppm and most preferably 130 to 150 ppm, of water.

4. The use according to any of claims 1 to 3, wherein the material is a thermal insulation material, preferably a thermal insulation material according to any of claims 5 to 9.

5. The use according to any of claims 1 to 3, wherein the material is a textile.

6. The use according to any of claims 1 to 3, wherein the material is a medical dressing or medical bandage.

## Patentansprüche

1. Verwendung einer Beschichtung, die Nanofilamente umfasst, die durch eine Polymerisationsreaktion einer oder mehrerer Silanverbindungen in Gegenwart von Wasser erhalten wurden, als flammhemmende Beschichtung, die auf einer Oberfläche eines Materials aufgebracht ist.

2. Verwendung gemäss Anspruch 1, wobei die eine oder mehreren Silanverbindungen ausgewählt sind aus Alkylsilanen, Alkenylsilanen, Arylsilanen, oder deren Derivate, insbesondere aus Silanverbindungen der Formel I:
RₐSi(R¹)ₙ(X¹)₃₋ₙ I
wobei Rₐ eine geradkettige oder verzweigte C₁₋₂₄ Alkylgruppe oder eine aromatische Gruppe ist, die durch eine einfache kovalente Bindung oder eine Abstandshaltereinheit mit dem Si-Atom verbunden ist,
wobei R¹ eine geradkettige oder verzweigte Kohlenwasserstoffgruppe mit 1 bis 6 Kohlenstoffatomen ist,
wobei X¹ ist eine hydrolysierbare Gruppe ist, und
n 0 oder 1 ist, mit der Maßgabe, dass X¹ die gleichen oder unterschiedliche Gruppen darstellen kann.

3. Verwendung gemäss Anspruch 1 oder 2, wobei die Polymerisationsreaktion einer oder mehrerer Silanverbindungen in Gegenwart von Wasser in der Gasphase durchgeführt wird, und die relative Luftfeuchtigkeit im Bereich von 20 % bis 80 %, vorzugsweise in liegt im Bereich von 30 % bis 60 %, und am meisten bevorzugt im Bereich von 30 % bis 50 % liegt, oder in flüssiger Phase in einem aprotischen Lösungsmittel, wie Toluol, durchgeführt wird, in Gegenwart von 5 bis 500 ppm Wasser, vorzugsweise 60 bis 250 ppm Wasser, besonders bevorzugt 75 bis 150 ppm Wasser und am meisten bevorzugt 130 bis 150 ppm Wasser.

4. Verwendung gemäss einem der vorhergehenden Ansprüche 1 bis 3 wobei es sich bei dem Material um ein Wärmedämmmaterial, vorzugsweise um ein Wärmedämmmaterial gemäss einem der Ansprüche 5 bis 6 handelt.

5. Verwendung gemäss einem der vorhergehenden Ansprüche 1 bis 3, wobei es sich bei dem Material um ein Textil handelt.

6. Verwendung gemäss einem der vorhergehenden Ansprüche 1 bis 3, wobei es sich bei dem Material um einen medizinischen Verband oder eine medizinische Bandage handelt.

## Revendications

1. Utilisation d'un revêtement comprenant des nanofilaments obtenus par une réaction de polymérisation d'un ou plusieurs composés silane en présence d'eau, en tant que revêtement ignifuge, disposé sur une surface d'un matériau.

2. L'utilisation selon la revendication 1, dans laquelle l'un ou plusieurs composés silane sont choisis parmi les alkylsilanes, les alcénylsilanes, les arylsilanes ou leurs dérivés, en particulier parmi les composés silanes de formule I :
RₐSi(R¹)ₙ(X¹)₃₋ₙ
où Rₐ représente un groupe alkyle en C₁₋₂₄ à chaine droite ou ramifiée ou un groupe aromatique qui est lié par une simple liaison covalente ou une unité d'espacement à l'atome de Si,
où R¹ est un groupe hydrocarbone à chaine droite ou ramifiée ayant 1 à 6 atomes de carbone,
X¹ est un groupe hydrolysable, et
n est égal à 0 ou 1, à condition que X¹ puisse représenter des groupes identiques ou différents.

3. L'utilisation selon la revendication 1 ou 2, dans laquelle la réaction de polymérisation d'un ou plusieurs composés silane en présence d'eau est réalisée en phase gazeuse et l'humidité relative se situe dans la plage de 20 % à 80 %, de préférence dans la plage de 30 % à 60 %, et plus de préférence dans la plage de 30 % à 50 %, ou est réalisée en phase liquide dans un solvant aprotique, tel que le toluène, en présence de 5 à 500 ppm, de préférence 60 à 250 ppm, plus de préférence 75 à 150 ppm, et tout particulièrement 130 à 150 ppm, d'eau.

4. L'utilisation selon l'une quelconque des revendications 1 à 3, dans lequel le matériau est un matériau d'isolation thermique, de préférence un matériau d'isolation thermique selon l'une quelconque des revendications 5 à 6.

5. L'utilisation selon l'une quelconque des revendications 1 à 3, dans lequel le matériau est un matériau textile.

6. L'utilisation selon l'une quelconque des revendications 1 à 3, dans laquelle le matériau est un pansement médical ou un bandage médical.
